# EUROPÄISCHE PATENTSCHRIFT

(11) **EP 2 440 643 B1**
(45) Veröffentlichungstag und Bekanntmachung des Hinweises auf die Patenterteilung: **11.10.2017**
(21) Anmeldenummer: 10713947.9
(22) Anmeldetag: 15.04.2010
(51) Int. Cl.: C11D 3/30, C11D 3/00, C11D 3/50, A61Q 15/00, A61K 8/41, A61L 9/01

(54) **DUFTGEBENDES WASCH-, REINIGUNGS- ODER PFLEGEMITTEL**
ODOR-IMPARTING DETERGENT, CLEANING OR CARE PRODUCT
PRODUIT DE LAVAGE, DE NETTOYAGE OU D'ENTRETIEN PARFUMÉ

(30) Priorität: 09.06.2009 DE 102009026854
(43) Veröffentlichungstag der Anmeldung: 18.04.2012
(73) Patentinhaber: Henkel AG & Co. KGaA, 40589 Düsseldorf (DE)
(72) Erfinder: HUCHEL, Ursula, 50670 Köln (DE); BAUER, Andreas, 41564 Kaarst (DE); SAUF, Silvia, 40235 Düsseldorf (DE)
(74) Vertreter: Viering, Jentschura & Partner mbB Patent- und Rechtsanwälte
(86) Internationale Anmeldenummer: PCT/EP2010/054919
(87) Internationale Veröffentlichungsnummer: WO 2010/142481

(56) Entgegenhaltungen:
- EP-A1- 1 787 689
- JP-A- 2007 209 464
- JP-A- 2007 291 540
- JP-A- 2007 296 062
- JP-A- 2007 300 963
- JP-A- 2008 295 877
- US-A1- 2003 158 079
- US-A1- 2008 305 063

## Beschreibung

Die vorliegende Erfindung betrifft ein Wasch-, Reinigungs- oder Pflegemittel, enthaltend Duftaldehyde und/oder Duftketone sowie 2-Amino-1,3-propandiol und/oder substituiertes 2-Amino-1,3-propandiol. Sie betrifft ferner ein Textilreinigungs- oder konditionierverfahren, bei welchem das zu reinigende Textil einer Textilwäsche unter Einsatz eines entsprechenden Wasch-, Reinigungs-oder Pflegemittels unterworfen wird. Sie betrifft die Verwendung eines solchen Mittels zum Reinigen und/oder Konditionieren von textilen Flächengebilden, insbesondere in einer automatischen Waschmaschine. Sie betrifft die Verwendung von 2-Amino-1,3-propandiol und/oder substituiertem 2-Amino-1,3-propandiol zur Verlängerung der Duftwirkung des Wasch-, Reinigungs-oder Pflegemittels sowie zur Erzielung eines lange anhaltenden Frischegeruches bei der Anwendung des Wasch-, Reinigungs- oder Pflegemittels.

Bei der Anwendung von Wasch-, Reinigungs- oder Pflegemitteln verfolgt der Verbraucher nicht nur das Ziel, die zu behandelnden Objekte zu waschen, reinigen oder pflegen, sondern er wünscht sich auch, dass die behandelten Objekte, wie z.B. Textilien, nach der Wäsche gut riechen mögen. Insbesondere aus diesem Grunde enthalten die meisten kommerziell verfügbaren Wasch-, Reinigungs- oder Pflegemittel Riechstoffe.

Beim Einsatz herkömmlicher Wasch-, Reinigungs- oder Pflegemittel bleibt nach der Anwendung, insbesondere nach dem Waschen, jedoch oft nur ein verhältnismäßig schwacher Duft auf dem behandelten Objekt, wie insbesondere der Wäsche, zurück. Daher besteht auf Verbraucherseite ein fortwährendes Bedürfnis nach Wasch-, Reinigungs- oder Pflegemitteln, welche eine verbesserte Objektbeduftung, insbesondere Textilbeduftung, ermöglichen, vor allem mit Blick auf die Duftintensität, insbesondere hinsichtlich eines Frischeempfindens. US2003/158079 A1 betrifft Zusammensetzungen mit verbesserte Duftintensität und offenbart Zusammensetzungen enthaltend ein Vorteilmittel , wie z.B. ein Duftaldehyd oder ein Duftketon, und ein primäres und/oder sekundäres Amin, wie z.B. ein Aminoalkohol. Die Aufgabe der vorliegenden Erfindung war es daher, Wasch-, Reinigungs- oder Pflegemittel bereitzustellen, welche eine verbesserte Duftintensität bei der Anwendung, insbesondere im Zusammenhang mit der Textilbehandlung, zeigen.

Diese Aufgabe wurde gelöst von einem Wasch-, Reinigungs- oder Pflegemittel, wie in Anspruch 1 beschrieben, enthaltend
(a) Duftaldehyde und/oder Duftketone sowie
(b) 2-Amino-1,3-propandiol und/oder substituiertes 2-Amino-1,3-propandiol gemäß der Formel (I) 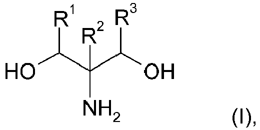
wobei in dieser Formel die Reste R¹, R² sowie R³, jeweils unabhängig voneinander, für Wasserstoff oder Kohlenwasserstoffreste stehen. R¹ und R³ stehen insbesondere, jeweils unabhängig voneinander, für C₁₋₆-Alkylreste, vorzugsweise C₁₋₃-Alkylreste, oder für Wasserstoff. Besonders bevorzugte Reste R² sind neben Wasserstoff insbesondere Methyl-, Ethyl-, und Hydroxymethylreste. In einer bevorzugten Ausführungsform bedeuten R¹, R² und R³ jeweils Wasserstoff.

Es konnte überraschend gefunden werden, dass die erfindungsgemäßen Wasch-, Reinigungs-oder Pflegemittel bei der Anwendung eine verbesserte Duftintensität zeigen, insbesondere im Zusammehang mit der Textilbehandlung. Z.B. konnte bei der Anwendung eines erfindungsgemäßen Wäschebehandlungsmittels, wie z.B. Waschmittel sowie Weichspüler, eine verbesserte Duftintensität der behandelten Wäsche gefunden werden. Dies gilt sowohl für die feuchte wie insbesondere auch für die trockene Wäsche. Hierbei wurde auch eine bessere Dauerhaftigkeit des Dufteindruckes gefunden, d.h. der gewünschte Dufteindruck hielt länger vor. Ferner weisen entsprechende Produkte eine besonders gute Lagerstabilität auf. Die erfindungsgemäßen Mittel ermöglichen es zudem, die Gesamtmenge an Parfüm, welche im Mittel enthalten ist, zu reduzieren, und dennoch Geruchsvorteile auf den gewaschenen Textilien zu erzielen, insbesondere mit Blick auf das Frischeempfinden.

In einer bevorzugten Ausführungsform der Erfindung zeichnet sich ein erfindungsgemäßes Mittel dadurch aus, dass die Komponente (a) und die Komponente (b) getrennt in die Wasch-, Reinigungs- oder Pflegemittelmatrix zugegeben werden. Bei dieser Vorgehensweise werden besonders vorteilhafte Duftintensitäten im erfindungsgemäßen Sinne ermöglicht. Es ist z.B. erfindungsgemäß möglich, die Komponente (a) mit der übrigen Parfümierung in das Produkt einzubringen und die Komponente (b) in einem gesonderten Schritt einzubringen. Weiterhin ist es auch erfindungsgemäß möglich, die Komponenten (a) und (b) in einem einzigen Schritt in das Produkt einzubringen, z.B. zusammen mit der übrigen Parfümierung, sofern die Komponenten (a) und (b) bei der Zugabe getrennt vorliegen, z.B. als separate Komponenten eines Parfümöls. In dem erfindungsgemäßen Mittel wird als Komponente (a) ein Duftaldehyd eingesetzt ausgewählt aus Adoxal, Anisaldehyd, Cymal, Ethylvanillin, Florhydral, Helional, Heliotropin, Hydroxycitronellal, Koavon, Lauraldehyd, Lyral, Methylnonylacetaldehyd, P. T. Bucinal, Phenylacetaldehyd, Undecylenaldehyd, Vanillin, 2,6,10-Trimethyl-9-undecenal, 3-Dodecen-1-al, alpha-n-Amylzimtaldehyd, 4-Methoxybenzaldehyd, Benzaldehyd, 3-(4-tert-Butylphenyl)-propanal, 2-Methyl-3-(para-methoxyphenyl)propanal, 2-Methyl-4-(2,6,6-timethyl-2(1)-cyclohexen-1-yl)butanal, 3-Phenyl-2-propenal, cis-/trans-3,7-Dimethyl- 2,6-octadien-1-al, 3,7-Dimethyl-6-octen-1-al, [(3,7-Dimethyl-6-octenyl)oxy]acetaldehyd, 4-Isopropylbenzyaldehyd, 1,2,3,4,5,6,7,8-Octahydro-8,8-dmethyl-2-naphthaldehyd, 2,4-Dimethyl-3-cyclohexen-1-carboxaldehyd, 2-Methyl-3-(isopropylphenyl)propanal, 1-Decanal, Decylaldehyd, 2,6-Dimethyl-5-heptenal, 4-(Tricyclo[5.2.1.0(2,6)]-decyliden-8)-butanal, Octahydro-4,7-methan-1H-indencarboxaldehyd, 3-Ethoxy-4-hydroxybenzaldehyd, para-Ethyl-alpha,alphadimethylhydrozimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methylphenylacetaldehyd, 7-Hydroxy-3,7-dimethyloctanal, Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al 2-Methylundecanal, 2-Methyldecanal, 1-Nonanal, 1-Octanal, 2,6,10-Trimethyl-5,9-undecadienal, 2-Methyl-3-(4-tert-butyl)propanal, Dihydrozimtaldehyd, 1-Methyl-4-(4-methyl-3-pentenyl)-3-cyclohexen-1-carboxaldehyd, 5- oder 6-Methoxyhexahydro-4,7-methanindan-1- oder -2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarboxaldehyd, 7-Hydroxy-3,7-dimethyl-octanal, trans-4-Decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarbox-aldehyd, 3,7-Dimethyl-2-methylen-6-octenal, Phenoxyacetaldehyd, 5,9-Dimethyl-4,8-decadienal, Päonienaldehyd (6,10-Dimethyl-3-oxa-5,9-undecadien-1-al), Hexahydro- 4,7-methanindan-1-carboxaldehyd, 2-Methyloctanal, alpha-Methyl-4-(1-methylethyl)benzolacetaldehyd, 6,6-Dimethyl-2-norpinen-2-propionaldehyd, para-Methylphenoxyacetaldehyd, 2-Methyl-3-phenyl-2-propen- 1-al 3,5,5-Trimethylhexanal, Hexahydro-8,8-dimethyl-2-naphthaldehyd, 3-Propylbicyclo[2.2.1]-hept-5-en-2-carbaldehyd, 9-Decenal, 3-Methyl-5-phenyl-1-pentanal, Methylnonylacetaldehyd, Hexanal, trans-2-Hexenal, 1-p-Menthen-q-carboxaldehyd oder Mischungen davon und/oder ein Duftketon ausgewählt aus Buccoxim; iso-Jasmon; Methyl-beta-naphthylketon; Moschusindanon; Tonalid/Musk plus; alpha-Damascon, beta-Damascon, delta-Damascon, iso-Damascon, Damascenon, Damarose, Methyldihydrojasmonat, Menthon, Carvon, Kampfer, Fenchon, alpha-Ionon, beta-Ionon, gamma-Methyl ge-nannt Ionon, Fleuramon, Dihydrojasmon, cis-Jasmon, iso-E-Super®, Methylcedrenylketon oder Methylcedry-Ion, Acetophenon, Methylacetophenon, para-Methoxyacetophenon, Methyl-beta-naphtylketon, Benzylaceton, Benzophenon, para-Hydroxyphenylbutanon, Sellerie-Keton oder Livescone, 6-Isopropyldecahydro-2-naphton, Dimethyloctenon, Frescomenthe, 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)yl)-1-propanon, 4-(4-Hydro-xy-3-methoxyphenyl)-2-butanon, 2-Acetyl-3,3-dimethylnorbornan, 6,7-Dihydro-1,1,2,3,3-pentamethyl-4(5H)in-danon, 4-Damascol, Dulcinyl oder Cassion, Gelson, Hexalon, Isocyclemon E, Methylcyclocitron, Methyllavendelketon, Orivon, para-tertiärem Butylcyclohexanon, Verdon, Delphon, Muscon, Neobutenon, Plicaton, Velou-ton, 2,4,4,7-Tetramethyl-oct-6-en-3-on, Tetrameran oder Mischungen davon. Bevorzugt können die Ketone ausgewählt sein aus Alpha Damascon, Delta Damascon, Iso Damascon, Carvon, Gamma-Methyl-ionon, Iso-E-Super, 2,4,4,7-Tetramethyl-oct-6-en-3-on, Benzylaceton, Beta Damascon, Damascenon, Methyldihydrojasmonat, Methyl-cedrylon, Hedion und Gemischen davon.

In einer weiteren bevorzugten Ausführungsform der Erfindung ist die Komponente (b) in Mengen von 0,001 Gew.-% bis 3 Gew.-%, vorzugsweise 0,01 bis 2 Gew.-%, insbesondere 0,1 bis 1 Gew.-% in dem Mittel enthalten, bezogen auf das gesamte Mittel. Auf diese Weise sind besonders gute Duftresultate im Sinne der Erfindung realisierbar. Wenn ein erfindungsgemäßes Mittel 0,001 Gew.-% bis 3 Gew.-%, vorzugsweise 0,01 bis 2 Gew.-%, insbesondere 0,1 bis 1 Gew.-% Duftaldehyde und/oder Duftketone enthält, Gew.-% bezogen auf das gesamte Mittel, so liegt ebenfalls eine bevorzugte Ausführungsform der Erfindung vor. So sind ebenfalls besonders gute Duftresultate im Sinne der Erfindung darstellbar. Für die Erzielung besonders guter Duftresultate beträgt das Verhältnis von Komponente (a) zu Komponente (b) 25:1 bis 1:5. Weiterhin ist es bevorzugt, dass die gesamte Riechstoffmenge des Mittels, umfassend also auch alle enthaltenen Duftaldehyde und/oder-ketone, 0,01 bis 10 Gew.-%, vorzugsweise 0,1 bis 5 Gew.-%, insbesondere 0,3 bis 3 Gew.-% beträgt. Dies entspricht einer bevorzugten Ausführungsform der Erfindung.

Gemäß einer weiteren bevorzugten Ausführungsform der Erfindung liegt das erfindungsgemäße Mittel in fester Form vor, vorzugsweise in Pulverform oder auch in Granulatform oder in Gestalt von Pressformkörpern, Z.B. Tabletten. Es ist aber auch möglich, dass das erfindungsgemäßes Mittel in flüssiger Form vorliegt, vorzugsweise in Gelform. Auch dies entspricht einer bevorzugten Ausführungsform der Erfindung.

Neben den Duftaldehyden und/oder Duftketonen sowie dem 2-Amino-1,3-propandiol und/oder substituierten 2-Amino-1,3-propandiol gemäß der obigen Formel (I) kann das erfindungsgemäße Mittel weitere geeignete Inhaltsstoffe umfassen. Das erfindungsgemäße Mittel enthält zumindest 5 Gew.-%, vorzugsweise zumindest 8 Gew.-%, insbesondere zumindest 10 Gew.-% Tensid, insbesondere Aniontensid und/oder Niotensid, und bis zu 50 Gew.-%. Eine sinnvolle Obergrenze für Tensid kann z.B. bei 40 Gew.-% oder 30 Gew.-% oder 20 Gew.-% liegen, Gew.-% jeweils bezogen auf das gesamte Mittel. Tenside werden weiter unten noch genauer beschrieben.

Ein weiterer Gegenstand der Erfindung ist ein Textilreinigungs- oder konditionierverfahren, bei welchem das zu reinigende Textil einer Textilwäsche unter Einsatz eines erfindungsgemäßen Wasch-, Reinigungs- oder Pflegemittels unterworfen wird, insbesondere in einer automatischen Waschmaschine, vorzugsweise bei Temperaturen nicht über 60°C, insbesondere nicht über 40°C. Ein weiterer Gegenstand der Erfindung liegt auch in der Verwendung eines erfindungsgemäßen Wasch-, Reinigungs- oder Pflegemittels zum Reinigen und/oder Konditionieren von textilen Flächengebilden, insbesondere in einer automatischen Waschmaschine, vorzugsweise bei Temperaturen nicht über 60°C, insbesondere nicht über 40°C.

Ein weiterer Gegenstand der Erfindung liegt in der Verwendung von 2-Amino-1,3-propandiol und/oder substituiertem 2-Amino-1,3-propandiol gemäß der Formel (I) 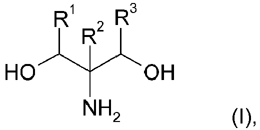 wobei in dieser Formel die Reste R¹, R² sowie R³, jeweils unabhängig voneinander, für Wasserstoff oder Kohlenwasserstoffreste stehen, als separat zugegebene Komponente in riechstoffhaltigen Wasch-, Reinigungs- oder Pflegemitteln, welche Duftstoffketone und/oder-aldehyde enthalten, zur Verlängerung der Duftwirkung des Wasch-, Reinigungs- oder Pflegemittels. "Separat zugegebene Komponente" bezieht sich hierbei auf die ebenfalls enthaltenen Duftstoffketone und/oder-aldehyde und soll bedeuten, dass 2-Amino-1,3-propandiol und/oder substituiertem 2-Amino-1,3-propandiol sowie die Duftstoffketone und/oder -aldehyde als gesonderte Entitäten in das Wasch-, Reinigungs- oder Pflegemittel eingehen. Es ist z.B. auch möglich, die Komponenten (a) und (b) in einem einzigen Schritt in das Produkt einzubringen, z.B. zusammen mit der übrigen Parfümierung, sofern die Komponenten (a) und (b) bei der Zugabe getrennt vorliegen, z.B. als separate Komponenten eines Parfümöls.

Ein weiterer Gegenstand der Erfindung liegt in der Verwendung von 2-Amino-1,3-propandiol und/oder substituiertem 2-Amino-1,3-propandiol gemäß der Formel (I) 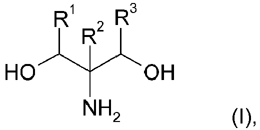 wobei in dieser Formel die Reste R¹, R² sowie R³, jeweils unabhängig voneinander, für Wasserstoff oder Kohlenwasserstoffreste stehen, als separat zugegebene Komponente in riechstoffhaltigen Wasch-, Reinigungs- oder Pflegemitteln, welche Duftstoffketone und/oder -aldehyde enthalten, zur Erzielung eines lange anhaltenden Frischegeruches bei der Anwendung des Wasch-, Reinigungs-oder Pflegemittels. "Separat zugegebene Komponente" ist hierbei wie zuvor beschrieben zu verstehen.

Bei dem Wasch-, Reinigungs- oder Pflegemittel kann es sich insbesondere um ein Textilbehandlungsmittel in Form eines Textilwaschmittels, Weichspülers, weichmachenden Waschmittels oder Waschhilfsmittels handeln. Ebenso kann es sich z.B. um ein Reinigungsmittel für harte Oberflächen handeln, wie vorzugsweise um ein Geschirrspülmittel, insbesondere um ein maschinelles Geschirrspülmittel. Ebenso kann es sich um Reinigungsmittel wie z.B. Haushaltsreiniger, Allzweckreiniger, Fensterreiniger, Fußbodenreiniger usw. handeln. Vorzugsweise kann es sich um ein Produkt zur Reinigung von WC-Becken und Urinalen handeln, vorteilhafterweise um einen Spülreiniger zum Einhängen in das WC-Becken oder den Spülkasten, insbesondere um einen sogenannten WC-Stein. Im Sinne des Pflegemittels handelt es sich bevorzugt um Kosmetika, wie z.B. Haarshampoos, Deos usw., welche für die Körperpflege und/oder -reinigung einsetzbar sind. Im Sinne des Pflegemittels kann es sich ferner auch um Air-Care-Produkte und Raumluftverbesserer handeln.

Gemäß einer bevorzugten Ausführungsform handelt es sich bei dem erfindungsgemäßen Mittel daher um ein Textilbehandlungsmittel, ein Bügelhilfsmittel, ein Reinigungstuch, ein Textilwaschmittel, einen Weichspüler, ein Reinigungsmittel, insbesondere für harte und/oder weiche Oberflächen, einen Haushaltsreiniger, ein Pflegemittel, ein Waschpflegemittel, ein Raumbeduftungsmittel, einen Luftverbesserer, ein Konditioniermittel, ein Färbemittel, einen Weichspüler, ein Konditioniersubstrat, ein Putzmittel, ein kosmetisches Mittel, ein Bleichmittel, ein Entkalkungsmittel, ein Autopflegemittel, Fußbodenpflegemittel, Herdpflegemittel, Lederpflegemittel, Möbelpflegemittel, ein Scheuermittel, ein Desinfektionsmittel, ein Beduftungsmittel, ein Schimmelentfernungsmittel und/oder ein Vorprodukt der vorgenannten Mittel. Es ist ein Vorteil der Erfindung, dass die erfindungsgemäßen Mittel sehr lagerstabil sind.

Nach einer weiteren bevorzugten Ausführungsform der Erfindung sind in dem erfindungsgemäßen Mittel zusätzliche(n) Duftstoff(e) enthalten, insbesondere ausgewählt aus der Gruppe umfassend Duftstoffe natürlichen oder synthetischen Ursprungs, bevorzugt leichter flüchtige Duftstoffe, höhersiedende Duftstoffe, feste Duftstoffe und/oder haftfeste Duftstoffe.

Haftfeste Riechstoffe, die im Rahmen der vorliegenden Erfindung mit Vorteil einsetzbar sind, sind beispielsweise etherische Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemon-grasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl.

Aber auch höhersiedende bzw. feste Riechstoffe natürlichen oder synthetischen Ursprungs können im Rahmen der vorliegenden Erfindung als haftfeste Riechstoffe bzw. Riechstoffgemische, also Duftstoffe eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, α-Bromstyrol, n-Decylaldehyd, n-Dodecylalde-hyd, Eugenof, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon- Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresol-methylether, Cumarin, p-Methoxyacetophenon, Methyl-n-amylketon, Methylanthranil-säure-methylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadekanolid, β-Phenylethylalkohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimatalkohol, Zimtsäure, Zimtsäureethylester, Zimtsäurebenzylester.

Zu den leichter flüchtigen Duftstoffen zählen insbesondere die niedriger siedenden Riechstoffe natürlichen oder synthetischen Ursprung, die allein oder in Mischungen eingesetzt werden können. Beispiele für leichter flüchtige Duftstoffe sind Alkylisothiocyanate (Alkylsenföle), Butandion, Limonen, Linalool, Linaylacetat und -Propionat, Menthol, Menthon, Methyl-n-heptenon, Phellandren, Phenylacetaldehyd, Terpinylacetat, Zitral, Zitronellal.

Zur beschleunigten oder verzögerten Freisetzung von Duftstoffen können alle im Stand der Technik bekannten Verfahren angewendet werden, soweit sie dem Fachmann als geeignet erscheinen. Gemäß einer bevorzugten Ausführungsform der Erfindung umfasst das erfindungsgemäße Mittel geträgerten und/oder verkapselten Duftstoff.

Nach einer weiteren bevorzugten Ausführungsform weist das erfindungsgemäße Wasch, Pflege-oder Reinigungsmittel, wenigstens eine, vorzugsweise mehrere, aktive Komponenten, insbesondere wasch-, pflege-, reinigungsaktive und/oder kosmetische Komponenten auf, vorteilhafterweise ausgewählt aus der Gruppe umfassend anionische Tenside, kationische Tenside, amphotere Tenside, nichtionische Tenside, Acidifizierungsmittel, Alkalisierungsmittel, Anti-Knitter-Verbindungen, antibakterielle Stoffe, Antioxidantien, Antiredepositionsmittel, Antistatika, Buildersubstanzen, Bleichmittel, Bleichaktivatoren, Bleichstabilisatoren, Bleichkatalysatoren, Bügelhilfsmittel, Cobuilder, Duftstoffe, Einlaufverhinderer, Elektrolyte, Enzyme, Farbschutzstoffe, Färbemittel, Farbstoffe, Farbübertragungsinhibitoren, Fluoreszensmittel, Fungizide, Germizide, geruchskomplexierende Substanzen, Hilfsmittel, Hydrotrope, Klarspüler, Komplexbildner, Konservierungsmittel, Korrosionsinhibitoren, wassermischbare organische Lösungsmittel, optische Aufheller, Parfüme, Parfümträger, Perlglanzgeber, pH-Stellmittel, Phobier- und Imprägniermittel, Polymere, Quell- und Schiebefestmittel, Schauminhibitoren, Schichtsilikate, schmutzabweisende Stoffe, Silberschutzmittel, Silikonöle, Soil release-Wirkstoffe, UV-Schutz-Substanzen, Viskositätsregulatoren, Verdickungsmittel, Verfärbungsinhibitoren, Vergrauungsinhibitoren, Vitamine und/oder Weichspüler. Im Sinne dieser Erfindung beziehen sich Angaben für das erfindungsgemäße Mittel in Gew.-%, wenn nicht anders angegeben, auf das Gesamtgewicht des erfindungsgemäßen Mittels.

Die Mengen der einzelnen Inhaltsstoffe in den erfindungsgemäßen Mitteln orientieren sich jeweils am Einsatzzweck der betreffenden Mittel und der Fachmann ist mit den Größenordnungen der einzusetzenden Mengen der Inhaltsstoffe vertraut oder kann diese der zugehörigen Fachliteratur entnehmen. Je nach Einsatzzweck der erfindungsgemäßen Mittel wird man beispielsweise den Tensidgehalt höher oder niedriger wählen. Üblicherweise kann z. B. der Tensidgehalt beispielsweise von Waschmitteln zwischen 10 und 50 Gew.-%, vorzugsweise zwischen 12,5 und 30 Gew.-% und insbesondere zwischen 15 und 25 Gew.-% betragen, während Reinigungsmittel für das maschinelle Geschirrspülen z.B. zwischen 0,1 und 10 Gew.-%, vorzugsweise zwischen 0,5 und 7,5 Gew.-% und insbesondere zwischen 1 und 5 Gew.-% Tenside enthalten können.

Die erfindungsgemäßen Mittel enthalten Tenside, wobei bevorzugt anionische Tenside, nichtionische Tenside und deren Gemische, aber auch kationische Tenside in Frage kommen. Geeignete nichtionische Tenside sind insbesondere Ethoxylierungs- und/oder Propoxylierungsprodukte von Alkylglykosiden und/oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Geeignete anionische Tenside sind insbesondere Seifen und solche, die Sulfat- oder SulfonatGruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Kationische Tenside werden vorzugsweise unter den Esterquats und/oder den quaternären Ammoniumverbindungen (QAV) gemäß der allgemeinen Formel (R^{I})(R^{II})(R^{III})(R^{IV})N⁺ X⁻ ausgewählt, in der R^{I} bis R^{IV} für gleiche oder verschiedene C₁₋₂₂-Alkylreste, C₇₋₂₈-Arylalkylreste oder heterozyklische Reste stehen, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, und X⁻ für Halogenidionen, Sulfationen, Hydroxidionen oder ähnliche Anionen steht. QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxysubstituierte Alkyl-Reste verfügen, sind wenig reaktiv und werden beispielsweise mit Dimethylsulfat quaterniert. In Frage kommende QAV sind beispielweise Benzalkoniumchlorid (N Alkyl-N,N dimethyl-benzylammoniumchlorid), Benzalkon B (m,p-Dichlorbenzyl-dimethyl-C₁₂-alkylammo-niumchlorid, Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid), Benzetoniumchlorid (N,N Dimethyl-N [2-[2-[p-(1,1,3,3-tetramethylbutyl)phenoxy]-ethoxy]-ethyl]-benzylammoniumchlorid), Dialkyldimethylammoniumchloride wie Di-n-decyl-dimethyl-ammoniumchlorid, Didecyldimethylammonium-bromid, Dioctyl-dimethyl-ammoniumchlorid, 1-Cetylpyridiniumchlorid und Thiazolinjodid sowie deren Mischungen. Bevorzugte QAV sind die Benzalkoniumchloride mit C₈-C₂₂-Alkylresten, insbesondere C₁₂-C₁₄-Alkyl-benzyl-dimethylammoniumchlorid.

Unter Esterquats sollen hier vorzugsweise Verbindungen der allgemeinen Formel IV, 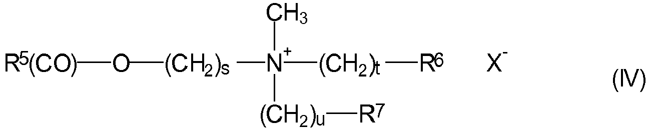 verstanden werden, in der R⁵ für einen Alkyl- oder Alkenylrest mit 12 bis 22 Kohlenstoffatomen und 0, 1, 2 oder 3 Doppelbindungen, R⁶ und R⁷ unabhängig voneinander für H, OH oder O(CO)R⁵, s, t und u jeweils unabhängig voneinander für den Wert 1, 2 oder 3 und X⁻ für ein Anion, insbesondere Halogenid, Methosulfat, Methophosphat oder Phosphat sowie Mischungen aus diesen, steht. Bevorzugt sind Verbindungen, die für R⁶ die Gruppe O(CO)R⁵ und für R⁵ einen Alkylrest mit 16 bis 18 Kohlenstoffatomen enthalten. Besonders bevorzugt sind Verbindungen, bei denen R⁷ zudem für OH steht. Beispiele für Verbindungen der Formel (IV) sind Methyl-N-(2-hydroxyethyl)-N,N-di(talgacyl-oxyethyl)ammonium-methosulfat, Bis-(palmitoyl)-ethyl-hydroxyethyl-methyl-ammonium-methosulfat oder Methyl-N,N-bis(acyloxyethyl)-N-(2-hydroxyethyl)ammonium-methosulfat. Werden quarternierte Verbindungen der Formel (IV) eingesetzt, die ungesättigte Gruppen aufweisen, sind die Acylgruppen bevorzugt, deren korrespondierende Fettsäuren eine Jodzahl zwischen 5 und 80, vorzugsweise zwischen 10 und 60 und insbesondere zwischen 15 und 45 aufweisen und/oder die ein cis/trans-Isomerenverhältnis (in Mol-%) von größer als 30 : 70, vorzugsweise größer als 50 : 50 und insbesondere größer als 70 : 30 haben. Handelsübliche Beispiele sind die von der Firma Stepan unter dem Warenzeichen Stepantex^{®} vertriebenen Methylhydroxyalkyldialkoyloxyalkylammoniummethosulfate oder die unter dem Handelsnamen Dehyquart^{®} bekannten Produkte der Firma Cognis Deutschland GmbH beziehungsweise die unter der Bezeichnung Rewoquat^{®} bekannten Produkte des Herstellers Goldschmidt-Witco.

Tenside sind in den erfindungsgemäßen Mitteln in Mengenanteilen von 5 Gew.-% bis 50 Gew.-%, insbesondere von 8 Gew.-% bis 30 Gew.-%, enthalten. Insbesondere in Wäschenachbehandlungsmitteln werden vorzugsweise bis zu 30 Gew.-%, insbesondere 5 Gew.-% bis 15 Gew.-% Tenside, unter diesen bevorzugt wenigstens anteilsweise Kationtenside, eingesetzt. Ein erfindungsgemäßes Mittel enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetra-kis(methylenphosphonsäure) und 1-Hydroxyethan-1,1-diphosphonsäure, polymere Hydroxyverbindungen wie Dextrin sowie polymere (Poly-)carbonsäuren, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative Molekülmasse der Homopolymeren ungesättiger Carbonsäuren liegt im allgemeinen zwischen 5 000 und 200 000, die der Copolymeren zwischen 2 000 und 200 000, vorzugsweise 50 000 bis 120 000, jeweils bezogen auf freie Säure. Ein besonders bevorzugtes Acrylsäure-Maleinsäure-Copolymer weist eine relative Molekülmasse von 50 000 bis 100 000 auf. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Als wasserlösliche organische Buildersubstanzen können auch Terpolymere eingesetzt werden, die als Monomere zwei ungesättigte Säuren und/oder deren Salze sowie als drittes Monomer Vinylalkohol und/ oder ein Vinylalkohol-Derivat oder ein Kohlenhydrat enthalten. Das erste saure Monomer beziehungsweise dessen Salz leitet sich von einer monoethylenisch ungesättigten C₃-C₈-Carbonsäure und vorzugsweise von einer C₃-C₄-Monocarbonsäure, insbesondere von (Meth)-acrylsäure ab. Das zweite saure Monomer beziehungsweise dessen Salz kann ein Derivat einer C₄-C₈-Dicarbonsäure sein, wobei Maleinsäure besonders bevorzugt ist. Die dritte monomere Einheit wird in diesem Fall von Vinylalkohol und/oder vorzugsweise einem veresterten Vinylalkohol gebildet. Insbesondere sind Vinylalkohol-Derivate bevorzugt, welche einen Ester aus kurzkettigen Carbonsäuren, beispielsweise von C₁-C₄-Carbonsäuren, mit Vinylalkohol darstellen. Bevorzugte Polymere enthalten dabei 60 Gew.-% bis 95 Gew.-%, insbesondere 70 Gew.-% bis 90 Gew.-% (Meth)acrylsäure bzw. (Meth)acrylat, besonders bevorzugt Acrylsäure bzw. Acrylat, und Maleinsäure bzw. Maleinat sowie 5 Gew.-% bis 40 Gew.-%, vorzugsweise 10 Gew.-% bis 30 Gew.-% Vinylalkohol und/oder Vinylacetat. Ganz besonders bevorzugt sind dabei Polymere, in denen das Gewichtsverhältnis von (Meth)acrylsäure beziehungsweise (Meth)acrylat zu Maleinsäure beziehungsweise Maleinat zwischen 1:1 und 4:1, vorzugsweise zwischen 2:1 und 3:1 und insbesondere 2:1 und 2,5:1 liegt. Dabei sind sowohl die Mengen als auch die Gewichtsverhältnisse auf die Säuren bezogen. Das zweite saure Monomer beziehungsweise dessen Salz kann auch ein Derivat einer Allylsulfonsäure sein, die in 2-Stellung mit einem Alkylrest, vorzugsweise mit einem C₁-C₄-Alkylrest, oder einem aromatischen Rest, der sich vorzugsweise von Benzol oder Benzol-Derivaten ableitet, substituiert ist. Bevorzugte Terpolymere enthalten dabei 40 Gew.-% bis 60 Gew.-%, insbesondere 45 bis 55 Gew.-% (Meth)acrylsäure beziehungsweise (Meth)acrylat, besonders bevorzugt Acrylsäure beziehungsweise Acrylat, 10 Gew.-% bis 30 Gew.-%, vorzugsweise 15 Gew.-% bis 25 Gew.-% Methallylsulfonsäure bzw. Methallylsulfonat und als drittes Monomer 15 Gew.-% bis 40 Gew.-%, vorzugsweise 20 Gew.-% bis 40 Gew.-% eines Kohlenhydrats. Dieses Kohlenhydrat kann dabei beispielsweise ein Mono-, Di-, Oligo- oder Polysaccharid sein, wobei Mono-, Di- oder Oligosaccharide bevorzugt sind. Besonders bevorzugt ist Saccharose. Durch den Einsatz des dritten Monomers werden vermutlich Sollbruchstellen in das Polymer eingebaut, die für die gute biologische Abbaubarkeit des Polymers verantwortlich sind. Diese Terpolymere weisen im Allgemeinen eine relative Molekülmasse zwischen 1 000 und 200 000, vorzugsweise zwischen 200 und 50 000 und insbesondere zwischen 3 000 und 10 000 auf. Weitere bevorzugte Copolymere sind solche, die als Monomere Acrolein und Acrylsäure/Acrylsäuresalze beziehungsweise Vinylacetat aufweisen. Die organischen Buildersubstanzen können, insbesondere zur Herstellung flüssiger Mittel, in Form wäßriger Lösungen, vorzugsweise in Form 30- bis 50-gewichtsprozentiger wäßriger Lösungen eingesetzt werden. Alle genannten Säuren werden in der Regel in Form ihrer wasserlöslichen Salze, insbesondere ihre Alkalisalze, eingesetzt.

Organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, erfindungsgemäßen Mitteln eingesetzt. Erfindungsgemäße Wäschenachbehandlungsmittel, wie z.B. Weichspüler, können gegebenenfalls auch frei von organischem Builder sein.

Als wasserlösliche anorganische Buildermaterialien kommen insbesondere Alkalisilikate und Polyphosphate, vorzugsweise Natriumtriphosphat, in Betracht. Als wasserunlösliche, wasserdispergierbare anorganische Buildermaterialien werden insbesondere kristalline oder amorphe Alkalialumosilikate, in Mengen von bis zu 50 Gew.-%, vorzugsweise nicht über 40 Gew.-% und in flüssigen Mitteln insbesondere von 1 Gew.-% bis 5 Gew.-%, eingesetzt. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm.

Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäßen Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁·yH₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl ß- als auch 5-Natriumdisilikate (Na₂Si₂O₅·yH₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in erfindungsgemäßen Mitteln eingesetzt werden. In einer weiteren bevorzugten Ausführungsform erfindungsgemäßer Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren bevorzugten Ausführungsform erfindungsgemäßer Mittel eingesetzt. Falls als zusätzliche Buildersubstanz auch Alkalialumosilikat, insbesondere Zeolith, vorhanden ist, beträgt das Gewichtsverhältnis Alumosilikat zu Silikat, jeweils bezogen auf wasserfreie Aktivsubstanzen, vorzugsweise 1:10 bis 10:1. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Buildersubstanzen sind in den erfindungsgemäßen Mitteln vorzugsweise in Mengen bis zu 60 Gew.-%, insbesondere von 5 Gew.-% bis 40 Gew.-%, enthalten. Erfindungsgemäße Wäschenachbehandlungsmittel, wie z.B. Weichspüler, sind vorzugsweise frei von anorganischem Builder.

Als geeignete Persauerstoffverbindungen kommen insbesondere organische Persäuren beziehungsweise persaure Salze organischer Säuren, wie Phthalimidopercapronsäure, Perbenzoesäure oder Salze der Diperdodecandisäure, Wasserstoffperoxid und unter den Anwendungsbedingungen Wasserstoffperoxid abgebende anorganische Salze, wie Perborat, Percarbonat und/oder Persilikat, in Betracht. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt wird Alkalipercarbonat, Alkaliperborat-Monohydrat oder insbesondere in flüssigen Mitteln Wasserstoffperoxid in Form wäßriger Lösungen, die 3 Gew.-% bis 10 Gew.-% Wasserstoffperoxid enthalten, eingesetzt. Falls ein erfindungsgemäßes Mittel Bleichmittel, wie vorzugsweise Persauerstoffverbindungen, enthält, sind diese in Mengen von vorzugsweise bis zu 50 Gew.-%, insbesondere von 5 Gew.-% bis 30 Gew.-%, vorhanden. Der Zusatz geringer Mengen bekannter Bleichmittelstabilisatoren wie beispielsweise von Phosphonaten, Boraten bzw. Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und Enolester sowie acetyliertes Sorbitol und Mannitol beziehungsweise deren Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, beispielsweise N-Benzoylcaprolactam. Hydrophil substituierte Acylacetale und Acyllactame werden ebenfalls bevorzugt eingesetzt. Auch Kombinationen konventioneller Bleichaktivatoren können eingesetzt werden. Derartige Bleichaktivatoren können im üblichen Mengenbereich, vorzugsweise in Mengen von 1 Gew.-% bis 10 Gew.-%, insbesondere 2 Gew.-% bis 8 Gew.-%, bezogen auf gesamtes Mittel, enthalten sein.

Zusätzlich zu den oben aufgeführten konventionellen Bleichaktivatoren oder an deren Stelle können auch Sulfonimine und/oder bleichverstärkende Übergangsmetallsalze bzw. Übergangsmetallkomplexe als sogenannte Bleichkatalysatoren enthalten sein. Zu den in Frage kommenden Übergangsmetallverbindungen gehören insbesondere Mangan-, Eisen-, Cobalt-, Ruthenium- oder Molybdän-Salenkomplexe und deren N-Analogverbindungen, Mangan-, Eisen-, Cobalt-, Ruthenium- oder Molybdän-Carbonylkomplexe, Mangan-, Eisen-, Cobalt-, Ruthenium-, Molybdän-, Titan-, Vanadium- und Kupfer-Komplexe mit stickstoffhaltigen Tripod-Liganden, Cobalt-, Eisen-, Kupfer- und Ruthenium-Amminkomplexe. Kombinationen aus Bleichaktivatoren und Übergangsmetall-Bleichkatalysatoren können ebenfalls eingesetzt werden. Bleichverstärkende Übergangsmetallkomplexe, insbesondere mit den Zentralatomen Mn, Fe, Co, Cu, Mo, V, Ti und/oder Ru, können in üblichen Mengen, vorzugsweise in einer Menge bis zu 1 Gew.%, insbesondere von 0,0025 Gew.-% bis 0,25 Gew.-% und besonders bevorzugt von 0,01 Gew.-% bis 0,1 Gew.-%, jeweils bezogen auf gesamtes Mittel, eingesetzt werden.

Als in den Mitteln verwendbare Enzyme kommen solche aus der Klasse der Proteasen, Cutinasen, Amylasen, Pullulanasen, Hemicellulasen, Cellulasen, Lipasen, Oxidasen und Peroxidasen sowie deren Gemische in Frage. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können an Trägerstoffen adsorbiert und/oder in Hüllsubstanzen eingebettet sein, um sie gegen vorzeitige Inaktivierung zu schützen. Sie sind in den erfindungsgemäßen Mitteln vorzugsweise nicht über 5 Gew.-%, insbesondere von 0,2 Gew.-% bis 2 Gew.-%, enthalten.

Die Mittel können als optische Aufheller beispielsweise Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls oder 4-(4-Chlorstyryl)-4'-(2-sulfostyryl)-diphenyls. Auch Gemische der vorgenannten Aufheller können verwendet werden.

Zu den geeigneten Schauminhibitoren gehören beispielsweise Organopolysiloxane und deren Gemische mit mikrofeiner, gegebenenfalls silanierter Kieselsäure sowie Pa-raffinwachse und deren Gemische mit silanierter Kieselsäure oder Bisfettsäure-alkylendiamiden. Mit Vorteilen werden auch Gemische aus verschiedenen Schaum-inhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche beziehungsweise dispergierbare Trägersubstanz gebunden. Insbesondere sind dabei Mischungen aus Paraffinwachsen und Bistearylethylendiamiden bevorzugt.

Zusätzlich können die Mittel auch Komponenten enthalten, welche die Öl- und Fettauswaschbarkeit aus Textilien positiv beeinflussen, sogenannte soil release-Wirkstoffe. Dieser Effekt wird besonders deutlich, wenn ein Textil verschmutzt wird, das bereits vorher mehrfach mit einem erfindungsgemäßen Mittel, das diese öl- und fettlösende Komponente enthält, gewaschen wurde. Zu den bevorzugten öl- und fettlösenden Komponenten zählen beispielsweise nichtionische Celluloseether wie Methylcellulose und Methylhydroxypropylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten mit monomeren und/oder polymeren Diolen, insbesondere Polymere aus Ethylenterephthalaten und/oder Polyethylenglykolterephthalaten oder anionisch und/oder nichtionisch modifizierten Derivaten von diesen.

Die Mittel können auch Farbübertragungsinhibitoren, vorzugsweise in Mengen von 0,1 Gew.-% bis 2 Gew.-%, insbesondere 0,1 Gew.-% bis 1 Gew.-%, enthalten, die in einer bevorzugten Ausgestaltung der Erfindung Polymere aus Vinylpyrrolidon, Vinylimidazol, Vinylpyridin-N-Oxid oder Copolymere aus diesen sind. Brauchbar sind sowohl Polyvinylpyrrolidone mit Molgewichten von 15 000 bis 50 000 wie auch Polyvinylpyrrolidone mit Molgewichten über 1 000 000, insbesondere von 1 500 000 bis 4 000 000, N-Vinylimidazol/N-Vinylpyrrolidon-Copolymere, Polyvinyloxazolidone, Copolymere auf Basis von Vinylmonomeren und Carbonsäureamiden, pyrrolidongruppenhaltige Polyester und Polyamide, gepfropfte Polyamidoamine und Polyethylenimine, Polymere mit Amidgruppen aus sekundären Aminen, Polyamin-N-Oxid-Polymere, Polyvinylalkohole und Copolymere auf Basis von Acrylamidoalkenylsulfonsäuren. Eingesetzt werden können aber auch enzymatische Systeme, umfassend eine Peroxidase und Wasserstoffperoxid beziehungsweise eine in Wasser Wasserstoffperoxid-liefernde Substanz. Der Zusatz einer Mediatorverbindung für die Peroxidase, zum Beispiel eines Acetosyringons, eines Phenolderivats oder eines Phenotiazins oder Phenoxazins, ist in diesem Fall bevorzugt, wobei auch zusätzlich obengenannte polymere Farbübertragungsinhibitorwirkstoffe eingesetzt werden können. Polyvinylpyrrolidon weist zum Einsatz in erfindungsgemäßen Mitteln vorzugsweise eine durchschnittliche Molmasse im Bereich von 10 000 bis 60 000, insbesondere im Bereich von 25 000 bis 50 000 auf. Unter den Copolymeren sind solche aus Vinylpyrrolidon und Vinylimidazol im Molverhältnis 5:1 bis 1:1 mit einer durchschnittlichen Molmasse im Bereich von 5 000 bis 50 000, insbesondere 10 000 bis 20 000 bevorzugt.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt.

Zu den in den erfindungsgemäßen Mitteln, insbesondere wenn sie in flüssiger oder pastöser Form vorliegen, verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den erfindungsgemäßen Mitteln vorzugsweise in Mengen von nicht über 30 Gew.-%, insbesondere von 6 Gew.-% bis 20 Gew.-%, vorhanden.

Zur Einstellung eines gewünschten, sich durch die Mischung der übrigen Komponenten nicht von selbst ergebenden pH-Werts können die erfindungsgemäßen Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind in den erfindungsgemäßen Mitteln vorzugsweise nicht über 20 Gew.-%, insbesondere von 1,2 Gew.-% bis 17 Gew.-%, enthalten.

Die Herstellung fester erfindungsgemäßer Mittel bereitet keine Schwierigkeiten und kann im Prinzip bekannter Weise, z.B. durch Sprühtrocknen oder Granulation, erfolgen, wobei optionale Persauerstoffverbindung und optionaler Bleichkatalysator ggf. später zugesetzt werden. Zur Herstellung erfindungsgemäßer Mittel mit erhöhtem Schüttgewicht, insbesondere im Bereich von 650 g/l bis 950 g/l, ist ein einen Extrusionsschritt aufweisendes Verfahren bevorzugt. Die Herstellung flüssiger erfindungsgemäßer Mittel bereitet ebenfalls keine Schwierigkeiten und kann ebenfalls in bekannter Weise erfolgen.

Gemäß einer bevorzugten Ausführungsform kann die erfindungsgemäße Lehre dazu eingesetzt werden, den Parfümanteil in Wasch-, Reinigungs- und Körperpflegemitteln signifikant herabzusetzen. Dadurch ist es möglich, parfümierte Produkte auch für solche besonders empfindlichen Konsumenten anzubieten, die normal parfümierte Produkte aufgrund spezieller Unverträglichkeiten und Irritationen nur eingeschränkt oder gar nicht verwenden können. In diesem Zusammenhang sind vor allem Hautpflegeprodukte und Deodorantien, aber auch Waschmittel, wie z.B. Handwaschmittel zu nennen.

Ein bevorzugtes erfindungsgemäßes festes, insbesondere pulverförmiges Waschmittel kann neben den erfindungsgemäßen Bestandteilen (also (a) Duftaldehyde und/oder Duftketone sowie (b) 2-Amino-1,3-propandiol und/oder substituiertes 2-Amino-1,3-propandiol gemäß der Formel (I)) insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5-30 Gew.-%
- Nichtionische Tenside, wie vorzugsweise Fettalkoholpolyglycolether, Alkylpolyglucosid, Fettsäureglucamid z.B. in Mengen von vorzugsweise 0,5-15 Gew.-%
- Gerüststoffe, wie z.B. Zeolith, Polycarboxylat, Natriumcitrat, in Mengen von z.B. 0-70 Gew.-% , vorteilhafterweise 5-60 Gew.-%, vorzugsweise 10-55 Gew.-%, insbesondere 15-40 Gew.-%,
- Alkalien, wie z.B. Natriumcarbonat, in Mengen von z.B. 0-35 Gew.-% vorteilhafterweise 1-30 Gew.-%, vorzugsweise 2-25 Gew.-%, insbesondere 5-20 Gew.-%,
- Bleichmittel, wie z.B. Natriumperborat, Natriumpercarbonat, in Mengen von z.B. 0-30 Gew.-% vorteilhafterweise 5-25 Gew.-%, vorzugsweise 10-20 Gew.-%,
- Korrosionsinhibitoren, z.B. Natriumsilicat, in Mengen von z.B. 0-10 Gew.-%, vorteilhafterweise 1-6 Gew.-%, vorzugsweise 2-5 Gew.-%, insbesondere 3-4 Gew.-%,
- Stabilisatoren, z.B. Phosphonate, vorteilhafterweise 0-1 Gew.-%,
- Schauminhibitor, z.B. Seife, Siliconöle, Paraffine vorteilhafterweise 0-4 Gew.-%, vorzugsweise 0,1-3 Gew.-%, insbesondere 0,2-1 Gew.-%,
- Enzyme, z.B. Proteasen, Amylasen, Cellulasen, Lipasen, vorteilhafterweise 0-2 Gew.-%, vorzugsweise 0,2-1 Gew.-%, insbesondere 0,3-0,8 Gew.-%,
- Vergrauungsinhibitor, z.B. Carboxymethylcellulose, vorteilhafterweise 0-1 Gew.-%,
- Verfärbungsinhibitor, z.B. Polyvinylpyrrolidon-Derivate, vorzugsweise 0-2 Gew.-%,
- Stellmittel, z.B. Natriumsulfat, vorteilhafterweise 0-20 Gew.-%,
- Optische Aufheller, z.B. Stilben-Derivat, Biphenyl-Derivat, vorteilhafterweise 0-0,4 Gew.-%, insbesondere 0,1-0,3 Gew.-%,
- ggf. weitere Duftstoffe
- ggf. Wasser
- ggf.Seife
- ggf.Bleichaktivatoren
- ggf.Cellulosderivate
- ggf.Schmutzabweiser,
Gew.-% jeweils bezogen auf das gesamte Mittel.

In einer anderen bevorzugten Ausführungsform der Erfindung liegt das Wasch-, Reinigungs- oder Pflegemittel in flüssiger Form vor, vorzugsweise in Gelform. Bevorzugte flüssige Wasch-, Reinigungs- oder Pflegemittel haben Wassergehalte von z.B. 10-95 Gew.-%, vorzugsweise 20-80 Gew.-% und insbesondere 30-70 Gew.-%, bezogen auf das gesamte Mittel. Im Falle von flüssigen Konzentraten kann der Wassergehalt auch besonders gering sein, z.B. < 30 Gew.-%, vorzugsweise < 20 Gew.-%, insbesondere < 15 Gew.-% betragen, Gew.-% jeweils bezogen auf das gesamte Mittel. Die flüssigen Mittel können auch nichtwässrige Lösungsmittel enthalten.

Ein bevorzugtes erfindungsgemäßes flüssiges, insbesondere gelförmiges Waschmittel kann neben den erfindungsgemäßen Bestandteilen insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5-40 Gew.-%
- Nichtionische Tenside, wie vorzugsweise Fettalkoholpolyglycolether, Alkylpolyglucosid, Fettsäureglucamid z.B. in Mengen von vorzugsweise 0,5-25 Gew.-%
- Gerüststoffe, wie z.B. Zeolith, Polycarboxylat, Natriumcitrat, vorteilhafterweise 0-15 Gew.-%, vorzugsweise 0,01-10 Gew.-%, insbesondere 0,1-5 Gew.-%,
- Schauminhibitor, z.B. Seife, Siliconöle, Paraffine, in Mengen von z.B. 0-10 Gew.-%, vorteilhafterweise 0,1-4 Gew.-%, vorzugsweise 0,2-2 Gew.-%, insbesondere 1-3 Gew.-%,
- Enzyme, z.B. Proteasen, Amylasen, Cellulasen, Lipasen, in Mengen von z.B. 0-3 Gew.-%, vorteilhafterweise 0,1-2 Gew.-%, vorzugsweise 0,2-1 Gew.-%, insbesondere 0,3-0,8 Gew.-%,
- Optische Aufheller, z.B. Stilben-Derivat, Biphenyl-Derivat, in Mengen von z.B. 0-1 Gew.-%, vorteilhafterweise 0,1-0,3 Gew.-%, insbesondere 0,1-0,4 Gew.-%,
- ggf. weitere Duftstoffe
- ggf. Stabilisatoren,
- Wasser
- ggf. Seife, in Mengen von z.B. 0-25 Gew.-%, vorteilhafterweise 1-20 Gew.-%, vorzugsweise 2-15 Gew.-%, insbesondere 5-10 Gew.-%,
- ggf. Lösungsmittel (vorzugsweise Alkohole), vorteilhafterweise 0-25 Gew.-%, vorzugsweise 1-20 Gew.-%, insbesondere 2-15 Gew.-%, Gew.-% jeweils bezogen auf das gesamte Mittel.

Ein bevorzugter erfindungsgemäßer flüssiger Weichspüler kann neben den erfindungsgemäßen Bestandteilen insbesondere noch Komponenten enthalten, die ausgewählt sind aus den folgenden:
- Kationische Tenside, wie insbesondere Esterquats, z.B. in Mengen von 5-30 Gew.-%,
- Cotenside, wie z.B. Glycerolmonostearat, Stearinsäure, Fettalkohole, Fettalkoholethoxylate, z.B. in Mengen von 0-5 Gew.-%, vorzugsweise 0,1-4 Gew.-%,
- Emulgatoren, wie z.B. Fettaminethoxylate, z.B. in Mengen von 0-4 Gew.-%, vorzugsweise 0,1-3 Gew.-%,
- ggf. weitere Duftstoffe
- Farbstoffe, vorzugsweise im ppm-Bereich
- Stabilisatoren, vorzugsweise im ppm-Bereich
- Lösemittel, wie insbesondere Wasser, in Mengen von vorzugsweise 60-90 Gew.-%,
- Gew.-% jeweils bezogen auf das gesamte Mittel.

### Beispiel:

Es wurde die Duftwirkung eines handelsüblichen teilchenförmigen Waschmittels untersucht, welches 0,4 Gew.-% 2,6-Dimethyl-5-Heptenal enthielt. Dies entspricht dem Waschmittel A. Ebenfalls wurde die Duftwirkung eines ansonsten vergleichbaren handelsüblichen teilchenförmigen Waschmittels untersucht, welches sowohl 0,4 Gew.-% 2,6-Dimethyl-5-Heptenal als auch 0,1 Gew.-% 2-Amino-1,3-propandiol enthielt. Dies entspricht dem Waschmittel B.

Zur Untersuchung der Duftwirkung der Waschmittel A und B wurden Waschversuche in einer handelsüblichen Waschmaschine (Miele Waschautomat Typ W 1734 WPS; Waschgut: Baumwolltextilien und Halbleinen in Menge von 3,0kg; Hauptwaschprogramm bei 40°C) durchgeführt. Die gewaschene Wäsche wurde anschließend einem Panel von 6 geruchlich geschulten Menschen vorgelegt, welche die Intensität des Wäscheduftes im feuchten Zustand sowie im trockenen Zustand (7 Tage nach der Wäsche) bewerteten. Zur Überprüfung des Wäscheduftes nach 7 Tagen wurde die feuchte Wäsche zuvor auf der Leine getrocknet und nach der Trocknung zusammengelegt und in einem offenen Regal aufbewahrt, bis 7 Tage seit der Wäsche vergangen waren.

Die Intensität wurde von jedem Bewerter auf einer Skala von 1 bis 5 bewertet, wobei der Wert 1 für einen nur schwach wahrnehmbaren Duft steht, während der Wert 5 für einen sehr intensiven Duft steht. Die Bewertung wurde zweimal wiederholt. Aus den gesamten Bewertungen wurde schließlich jeweils der gemittelte Wert berechnet. Es ergaben sich folgende gemittelte Werte:

| | Duftintensität der feuchten Wäsche | Duftintensität der trockenen Wäsche nach 7 Tagen |
|---|---|---|
| Waschmittel A | 3,3 | 1,8 |
| Waschmittel B | 4,7 | 4,5 |

Das mit der Erfindung im Einklang stehende Waschmittel B führte folglich zu einer verbesserten Duftintensität, sowohl bei der feuchten Wäsche wie insbesondere auch bei der trockenen Wäsche nach 7 Tagen.

## Patentansprüche

1. Wasch-, Reinigungs- oder Pflegemittel, enthaltend
(a) Duftaldehyde ausgewählt aus Adoxal, Anisaldehyd, Cymal, Ethylvanillin, Florhydral, Helional, Heliotropin, Hydroxycitronellal, Koavon, Lauraldehyd, Lyral, Methylnonylacetaldehyd, P.T. Bucinal, Phenylacetaldehyd, Undecylenaldehyd, Vanillin, 2,6,10-Trimethyl-9-undecenal, 3-Dodecen-1-al, alpha-n-Amylzimtaldehyd, 4-Methoxybenzaldehyd, Benzaldehyd, 3-(4-tert-Butylphenyl)-propanal, 2-Methyl-3-(para-methoxyphenyl)propanal, 2-Methyl-4-(2,6,6-timethyl-2(1)-cyclohexen-1-yl)butanal, 3-Phenyl-2-propenal, cis-/trans-3,7-Dimethyl-2,6-octadien-1-al, 3,7-Dimethyl-6-octen-1-al, [(3,7-Dimethyl-6-octenyl)oxy]acetaldehyd, 4-Isopropylbenzylaldehyd, 1,2,3,4,5,6,7,8-Octahydro-8,8-dmethyl-2-naphthaldehyd, 2,4-Dimethyl-3-cyclohexen-1-carboxaldehyd, 2-Methyl-3-(isopropylphenyl)propanal, 1-Decanal, Decylaldehyd, 2,6-Dimethyl-5-heptenal, 4-(Tricyclo[5.2.1.0(2,6)]-decyliden-8)-butanal, Octahydro-4,7-methan-1H-indencarboxaldehyd, 3-Ethoxy-4-hydroxybenzaldehyd, para-Ethyl-alpha,alpha-dimethylhydrozimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methylphenylacetaldehyd, 7-Hydroxy-3,7-dimethyloctanal, Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al, 2-Methylundecanal, 2-Methyldecanal, 1-Nonanal, 1-Octanal, 2,6,10-Trimethyl-5,9-undecadienal, 2-Methyl-3-(4-tert-butyl)propanal, Dihydrozimtaldehyd, 1-Methyl-4-(4-methyl-3-pentenyl)-3-cyclohexen-1-carboxaldehyd, 5- oder 6-Methoxyhexahydro-4,7-methanindan-1- oder -2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarboxaldehyd, 7-Hydroxy-3,7-dimethyl-octanal, trans-4-Decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarboxaldehyd, 3,7-Dimethyl-2-methylen-6-octenal, Phenoxyacetaldehyd, 5,9-Dimethyl-4,8-decadienal, Päonienaldehyd (6,10-Dimethyl-3-oxa-5,9-undecadien-1-al), Hexahydro-4,7-methanindan-1-carboxaldehyd, 2-Methyloctanal, alpha-Methyl-4-(1-methylethyl)benzolacetaldehyd, 6,6-Dimethyl-2-norpinen-2-propionaldehyd, para-Methylphenoxyacetaldehyd, 2-Methyl-3-phenyl-2-propen-1-al, 3,5,5-Trimethylhexanal, Hexahydro-8,8-dimethyl-2-naphthaldehyd, 3-Propylbicyclo[2.2.1]-hept-5-en-2-carbaldehyd, 9-Decenal, 3-Methyl-5-phenyl-1-pentanal, Methylnonylacetaldehyd, Hexanal, trans-2-Hexenal, 1-p-Menthen-q-carboxaldehyd oder Mischungen davon und/oder
Duftketone ausgewählt aus Buccoxim; iso-Jasmon; Methyl-beta-naphthylketon; Moschusindanon; Tonalid/Musk plus; alpha-Damascon, beta-Damascon, delta-Damascon, iso-Damascon, Damascenon, Damarose, Methyldihydrojasmonat, Menthon, Carvon, Kampfer, Fenchon, alpha-Ionon, beta-Ionon, gamma-Methyl genannt Ionon, Fleuramon, Dihydrojasmon, cis-Jasmon, iso-E-Super®, Methylcedrenylketon oder Methylcedrylon, Acetophenon, Methylacetophenon, para-Methoxyacetophenon, Methyl-beta-naphtylketon, Benzylaceton, Benzophenon, para-Hydroxyphenylbutanon, Sellerie-Keton oder Livescone, 6-Isopropyldecahydro-2-naphton, Dimethyloctenon, Frescomenthe, 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)yl)-1-propanon, 4-(4-Hydro-xy-3-methoxyphenyl)-2-butanon, 2-Acetyl-3,3-dimethylnorbornan, 6,7-Dihydro-1,1,2,3,3-pentamethyl-4(5H)in-danon, 4-Damascol, Dulcinyl oder Cassion, Gelson, Hexalon, Isocyclemon E, Methylcyclocitron, Methyllavendelketon, Orivon, para-tertiärem Butylcyclohexanon, Verdon, Delphon, Muscon, Neobutenon, Plicaton, Velouton, 2,4,4,7-Tetramethyl-oct-6-en-3-on, Tetrameran oder Mischungen davon sowie
(b) 2-Amino-1,3-propandiol und/oder substituiertes 2-Amino-1,3-propandiol gemäß der Formel (I) 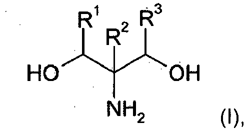 wobei in dieser Formel die Reste R¹, R² sowie R³, jeweils unabhängig voneinander, für Wasserstoff oder Kohlenwasserstoffreste stehen, wobei besonders bevorzugte Reste R² neben Wasserstoff insbesondere Methyl-, Ethyl-, und Hydroxymethylreste sind und
(c) 5 Gew.-% bis 50 Gew.-% Tenside, wobei
das Verhältnis von Komponente (a) zu Komponente (b) 25:1 bis 1:5 beträgt.

2. Mittel gemäß Anspruch 1, **dadurch gekennzeichnet, dass** die Komponente (a) und die Komponente (b) getrennt in die Wasch-, Reinigungs- oder Pflegemittelmatrix zugegeben werden.

3. Mittel gemäß einem der Ansprüche 1 oder 2, **dadurch gekennzeichnet, dass** die Komponente (b) in Mengen von 0,001 Gew.-% bis 3 Ges:-% in dem Mittel enthalten ist, Gew.-% bezogen auf das gesamte Mittel.

4. Mittel gemäß einem der Ansprüche 1 bis 3, **dadurch gekennzeichnet, dass** es 0,001 Gew.-% bis 3 Gew.-% Duftaldehyde und/oder Duftketone enthält, Gew.-% bezogen auf das gesamte Mittel.

5. Mittel gemäß einem der Ansprüche 1 bis 4, **dadurch gekennzeichnet, dass** die gesamte Riechstoffmenge des Mittels 0,01 bis 10 Gew.-% beträgt, Gew.-% bezogen auf das gesamte Mittel.

6. Mittel nach einem der Ansprüche 1-5, **dadurch gekennzeichnet, dass** es in fester Form vorliegt, vorzugsweise in Pulverform.

7. Mittel nach einem der Ansprüche 1-6, **dadurch gekennzeichnet, dass** es in flüssiger Form vorliegt, vorzugsweise in Gelform.

8. Mittel nach einem der Ansprüche 1-7, **dadurch gekennzeichnet, dass** es zumindest 5 Gew.-%, vorzugsweise zumindest 8 Gew.-%, insbesondere zumindest 10 Gew.-% Tensid enthält, insbesondere Aniontensid und/oder Niotensid, Gew.-% bezogen auf das gesamte Mittel

9. Textilreinigungs- oder konditionierverfahren, bei welchem das zu reinigende Textil einer Textilwäsche unter Einsatz eines Wasch-, Reinigungs- oder Pflegemittels nach einem der Ansprüche 1-8 unterworfen wird, insbesondere in einer automatischen Waschmaschine.

10. Verwendung eines Wasch-, Reinigungs- oder Pflegemittels nach einem der Ansprüche 1-8 zum Reinigen und/oder Konditionieren von textilen Flächengebilden, insbesondere in einer automatischen Waschmaschine.

11. Verwendung von 2-Amino-1,3-propandiol und/oder substituiertem 2-Amino-1,3-propandiol gemäß der Formel (I) 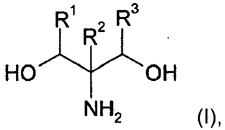 wobei in dieser Formel die Reste R¹, R² sowie R³, jeweils unabhängig voneinander, für Wasserstoff oder Kohlenwasserstoffreste stehen,
als separat zugegebene Komponente in riechstoffhaltigen Wasch-, Reinigungs- oder Pflegemitteln, welche Duftstoffketone und/oder -aldehyde enthalten, zur Verlängerung der Duftwirkung des Wasch-, Reinigungs- oder Pflegemittels.

12. Verwendung von 2-Amino-1,3-propandiol und/oder substituiertem 2-Amino-1,3-propandiol gemäß der Formel (I) 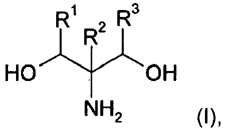 wobei in dieser Formel die Reste R¹, R² sowie R³, jeweils unabhängig voneinander, für Wasserstoff oder Kohlenwasserstoffreste stehen,
als separat zugegebene Komponente in riechstoffhaltigen Wasch-, Reinigungs- oder Pflegemitteln, welche Duftstoffketone und/oder -aldehyde enthalten, zur Erzielung eines lange anhaltenden Frischegeruches bei der Anwendung des Wasch-, Reinigungs- oder Pflegemittels.

## Claims

1. A washing, cleaning or care agent, containing
(a) fragrance aldehydes, selected from adoxal, anisaldehyde, cymal, ethylvanillin, Florhydral, helional, heliotropin, hydroxycitronellal, Koavone, lauraldehyde, Lyral, methyl nonyl acetaldehyde, P.T. Bucinal, phenylacetaldehyde, undecyl aldehyde, vanillin, 2,6,10-trimethyl-9-undecenal, 3-dodecen-1-al, alpha-n-amylcinnamaldehyde, 4-methoxybenzaldehyde, benzaldehyde, 3-(4-tert-butylphenyl)-propanal, 2-methyl-3-(paramethoxyphenyl propanal), 2-methyl-4-(2,6,6-trimethyl-2(1)-cyclohexene-1-yl)butanal, 3-phenyl-2-propenal, cis-/trans-3,7-dimethyl-2,6-octadien-1-al, 3,7-dimethyl-6-octen-1-al, [(3,7-dimethyl-6-octenyl)oxy]acetaldehyde, 4-isopropylbenzaldehyde, 1,2,3,4,5,6,7,8-octahydro-8,8-dimethyl-2-naphthaldehyde, 2,4-dimethyl-3-cyclohexene-1-carboxaldehyde, 2-methyl-3-(isopropylphenyl) propanal, 1-decanal, decylaldehyde, 2,6-dimethyl-5-heptenal, 4-(tricyclo[5.2.1.0(2,6)]-decylidene-8)-butanal, octahydro-4,7-methano-1H-indene carboxaldehyde, 3-ethoxy-4-hydroxybenzaldehyde, para-ethyl-alpha,alpha-dimethyl-hydrocinnamaldehyde, alpha-methyl-3,4-(methylenedioxy)-hydrocinnamaldehyde, 3,4-(methylenedioxy)benzaldehyde, alpha-n-hexylcinnamaldehyde, m-cymene-7-carboxaldehyde, alpha-methyl phenyl acetaldehyde, 7-hydroxy-3,7-dimethyloctanal, undecenal, 2,4,6-trimethyl-3-cyclohexene-1-carboxaldehyde, 4-(3)(4-methyl-3-pentenyl)-3-cyclohexene carboxaldehyde, 1-dodecanal, 2,4-dimethylcyclohexene-3-carboxaldehyde, 4-(4-hydroxy-4-methylpentyl)-3-cylohexene-1-carboxaldehyde, 7-methoxy-3,7-dimethyloctan-1-al, 2-methylundecanal, 2-methyldecanal, 1-nonanal, 1-octanal, 2,6,10-trimethyl-5,9-undecadienal, 2-methyl-3-(4-tert-butyl)propanal, dihydrocinnamaldehyde, 1-methyl-4-(4-methyl-3-pentenyl)-3-cyclohexene-1-carboxaldehyde, 5- or 6-methoxyhexahydro-4,7-methanindan-1- or -2-carboxaldehyde, 3,7-dimethyloctan-1-al, 1-undecanal, 10-undecen-1-al, 4-hydroxy-3-methoxybenzaldehyde, 1-methyl-3-(4-methylpentyl)-3-cyclohexene carboxyaldehyde, 7-hydroxy-3,7-dimethyloctanal, trans-4-decenal, 2,6-nonadienal, para-tolyl acetaldehyde, 4-methylphenylacetaldehyde, 2-methyl-4-(2,6,6-trimethyl-1-cyclohexene-1-yl)-2-butenal, ortho-methoxycinnamaldehyde, 3,5,6-trimethyl-3-cyclohexene carboxaldehyde, 3,7-dimethyl-2-methylene-6-octenal, phenoxyacetaldehyde, 5,9-dimethyl-4,8-decadienal, peony aldehyde (6,10-dimethyl-3-oxa-5,9-undecadien-1-al), hexahydro-4,7-methanoindan-1-carboxaldehyde, 2-methyloctanal, alpha-methyl-4-(1-methylethyl)benzeneacetaldehyde, 6,6-Dimethyl-2-norpinene-2-propionaldehyde, para-methyl phenoxy acetaldehyde, 2-methyl-3-phenyl-2-propen-1-al, 3,5,5-trimethylhexanal, hexahydro-8,8-dimethyl-2-naphthaldehyde, 3-propyl-bicyclo[2.2.1]-hept-5-ene-2-carbaldehyde, 9-decenal, 3-methyl-5-phenyl-1-pentanal, methyl nonyl acetaldehyde, hexanal, trans-2-hexenal, 1-p-menthene-q-carboxaldehyde or mixtures thereof, and/or
fragrance ketones selected from Buccoxime; iso jasmone; methyl beta-naphthyl ketone; musk indanone; tonalide/Musk Plus; alpha-damascone, beta-damascone, delta-damascone, iso damascone, damascenone, damarose, methyl dihydrojasmonate, menthone, carvone, camphor, fenchone, alpha-ionone, beta-ionone, gamma-methyl referred to as ionone, dihydrojasmone, cis-jasmone, iso-E-Super®, methyl cedryl ketone or methyl cedrylone, acetophenone, methylacetophenone, para-methoxyacetophenone, methyl-beta-naphtyl ketone, benzyl acetone, benzophenone, para-hydroxyphenyl butanone, celery ketone or livescone, 6-isopropyldecahydro-2-naphthol, dimethyl octenone, frescomenthe, 4-(1-ethoxyvinyl)-3,3,5,5-tetramethylcyclohexanone, methyl heptenone, 2-(2-(4-methyl-3-cyclohexene-1-yl)propyl)cyclopentan one, 1-(p-menthene-6(2)-yl)-1-propanone, 4-(4-hydroxy-3-methoxyphenyl)-2-butanone, 2-acetyl-3,3-dimethyl-norbornane, 6,7-dihydro-1,1,2,3,3-pentamethyl-4(5H)-indanone, 4-damascol, Dulcinyl or Cassione, gelsone, hexalone, isocyclemone E, methyl cyclocitrone, methyl lavender ketone, orivone, para-tertiary butylcyclohexanone, verdone, delphone, muscone, neobutenone, plicatone, veloutone, 2,4,4,7-tetramethyl-oct-6-en-3-one, tetrameran or mixtures thereof, and
(b) 2-amino-1,3-propanediol and/or substituted 2-amino-1,3-propanediol according to formula (I), 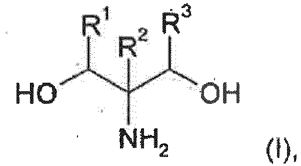 wherein, in this formula, the functional groups R¹, R² and R³ represent, independently of one another in each case, hydrogen or hydrocarbon functional groups, wherein particularly preferred functional groups R² are, in addition to hydrogen, in particular methyl-, ethyl- and hydroxymethyl functional groups, and
(c) 5 wt.% to 50 wt.% surfactants, wherein
the ratio of component (a) to component (b) is from 25:1 to 1:5.

2. The agent according to claim 1, **characterized in that** component (a) and component (b) are added separately to the washing, cleaning or care agent matrix.

3. The agent according to one of claims 1 or 2, **characterized in that** component (b) is contained in the agent in amounts of from 0.001 wt.% to 3 wt.%, wt.% being based on the total agent.

4. The agent according to one of claims 1 to 3, **characterized in that** it contains from 0.001 wt.% to 3 wt.% fragrance aldehydes and/or fragrance ketones, wt.% being based on the total agent.

5. The agent according to one of claims 1 to 4, **characterized in that** the total amount of odorant of the agent is from 0.01 to 10 wt.%, wt.% being based on the total agent.

6. The agent according to one of claims 1 to 5, **characterized in that** it is in solid form, preferably in powder form.

7. The agent according to one of claims 1 to 6, **characterized in that** it is in liquid form, preferably in gel form.

8. The agent according to one of claims 1 to 7, **characterized in that** it contains at least 5 wt.%, preferably at least 8 wt.%, in particular at least 10 wt.% surfactant, in particular anionic surfactant and/or non-ionic surfactant, wt.% being based on the total agent.

9. A method for cleaning or conditioning textiles, wherein the textile to be cleaned is subjected to a textile-washing process using a washing, cleaning or care agent according to one of claims 1 to 8, in particular in an automatic washing machine.

10. The use of a washing, cleaning or care agent according to one of claims 1 to 8 for cleaning and/or conditioning textile fabrics, in particular in an automatic washing machine.

11. The use of 2-amino-1,3-propanediol and/or substituted 2-amino-1,3-propanediol according to formula (I), 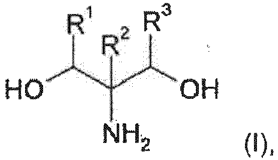 wherein, in this formula, the functional groups R¹, R² and R³ represent, independently of one another in each case, hydrogen or hydrocarbon functional groups,
as a component that is added separately to odorant-containing washing, cleaning or care agents that contain fragrance ketones and/or aldehydes, in order to extend the fragrance effect of the washing, cleaning or care agent.

12. The use of 2-amino-1,3-propanediol and/or substituted 2-amino-1,3-propanediol according to formula (I), 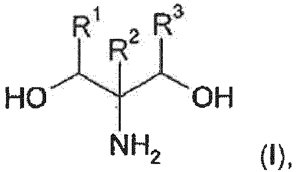 wherein, in this formula, the functional groups R¹, R² and R³ represent, independently of one another in each case, hydrogen or hydrocarbon functional groups,
as a component that is added separately to odorant-containing washing, cleaning or care agents that contain fragrance ketones and/or aldehydes, in order to achieve a long-lasting fresh scent when using the washing, cleaning or care agent.

## Revendications

1. Produit de lavage, de nettoyage ou d'entretien contenant
(a) des aldéhydes parfumés sélectionnés parmi l'adoxal, l'anisaldéhyde, le cymal, l'éthylvanilline, le florhydral, l'hélional, l'héliotropine, l'hydroxycitronellal, la koavone, le lauraldéhyde, le lyral, le méthylnonylacétaldéhyde, le P.T. bucinal, le phénylacétaldéhyde, l'undécylènealdéhyde, la vanilline, le 2,6,10-triméthyl-9-undécénal, le 3-dodécène-1-al, l'alpha-n-amylzimtaldéhyde, le 4-méthoxybenzaldéhyde, le 3-(4-tert-butylphényl)-propanal, le 2-méthyl-3-(para-méthoxyphényl)propanal, le 2-méthyl-4-(2,6,6-timéthyl-2(1)-cyclohexène-1-yl)butanal, le 3-phényl-2-propenal, le cis-/trans-3,7-diméthyl-2,6-octadiène-1-al, le 3,7-diméthyl-6-octène-1-al, le [(3,7-diméthyl-6-octényl)oxy]acétaldéhyde, le 4-isopropylbenzylaldéhyde, le 1,2,3,4,5,6,7,8-octahydro-8,8-dméthyl-2-naphtaldéhyde, le 2,4-diméthyl-3-cyclohexène-1-carboxaldéhyde, le 2-méthyl-3-(isopropylphényl)propanal, le 1-décanal, le décylaldéhyde, le 2,6-diméthyl-5-heptenal, le 4-(tricyclo[5.2.1.0(2,6)]-decylidène-8)-butanal, l'octahydro-4,7-méthane-1H-indencarboxaldéhyde, le 3-éthoxy-4-hydroxybenzaldéhyde, le para-éthyl-alpha,alpha-diméthylhydrozimtaldéhyde, l'alpha-méthyl-3,4-(méthylènedioxy)-hydrozimtaldéhyde, le 3,4-méthylènedioxybenzaldéhyde, l'alpha-n-hexylzimtaldéhyde, le m-cymène-7-carboxaldéhyde, l'alpha-méthylphénylacétaldéhyde, le 7-hydroxy-3,7-diméthyloctanal, l'undécénal, le 2,4,6-triméthyl-3-cyclohexène-1-carboxaldéhyde, le 4-(3)(4-méthyl-3-pentényl)-3-cyclohexènecarboxaldéhyde, le 1-dodécanal, le 2,4-diméthylcyclohexène-3-carboxaldéhyde, le 4-(4-hydroxy-4-méthylpentyl)-3-cyclohexène-1-carboxaldéhyde, le 7-méthoxy-3,7-diméthyloctane-1-al, le 2-méthylundécanal, le 2-méthyldécanal, le 1-nonanal, le 1-octanal, le 2,6,10-triméthyl-5-9-undécadiénal, le 2-méthyl-3-(4-tert-butyl)propanal, le dihydrozimtaldéhyde, le 1-méthyl-4-(4-méthyl-3-penényl)-3-cyclohexène-1-carboxaldéhyde, le 5- ou 6-méthoxyhexahydro-4,7-méthaneindane-1- ou -2-carboxaldéhyde, le 3,7-diméthyloctane-1-al, le 1-undécanal, le 10-undécène-1-al, le 4-hydroxy-3-méthoxybenzaldéhyde, le 1-méthyl-3-(4-méthylpentyl)-3-cyclohexènecarboxaldéhyde, le 7-hydroxy-3,7-diméthyl-octanal, le trans-4-décénal, le 2,6-nonadiénal, le para-tolylacétaldéhyde, le 4-méthylphénylacétaldéhyde, le 2-méthyl-4-(2,6,6-triméthyl-1-cyclohexène-1-yl)-2-buténal, l'ortho-méthoxyzimtaldéhyde, le 3,5,6-triméthyl-3-cyclohexènecarboxaldéhyde, le 3,7-diméthyl-2-méthylène-6-octénal, le phénoxyacétaldéhyde, le 5,9-diméthyl-4,8-décadiénal, l'aldéhyde de pivoine (6,10-diméthyl-3-oxa-5,9-undécadiène-1-al), l'hexahydro-4,7-méthaneindane-1-carboxaldéhyde, le 2-méthyloctanal, l'alpha-méthym-4-(1-méthyléthyl)benzolacétaldéhyde, le 6,6-diméthyl-2-norpinène-2-propionaldéhyde, le para-méthylphénoxyacétaldéhyde, le 2-méthyl-3-phényl-2-propène-1-al, le 3,5,5-triméthylhexanal, l'hexahydro-8,8-diméthyl-2-naphtaldéhyde, le 3-propylbicyclo[2.2.1]-hept-5-en-2-carbaldéhyde, le 9-décénal, le 3-méthyl-5-phényl-1-pentanal, le méthylnonylacétaldéhyde, l'hexanal, le trans-2-hexénal, le 1-p-menthène-q-carboxaldéhyde ou des composés de ceux-ci et/ou des cétones parfumés sélectionnés parmi le buccoxime ; l'iso-jasmone ; la cétone méthyl-bêta-naphtylique ; l'indanone de musc ; la tonalide/musc plus ; l'alpha-damascone, la bêta-damascone, la delta-damascone, l'iso-damascone, la damascénone, la damarose, le dihydrojasmonate de méthyle, la menthone, la carvone, le camphre, la fenchone, l'alpha-ionone, la bêta-ionone, le gamma-méthyl appelé ionone, la fleuramone, la dihydrojasmone, la cis-jasmone, l'iso-E-Super®, la méthyl cédrényl cétone ou la méthyl cédrylone, l'acétophénone, la méthylacétophénone, la para-méthoxyacétophénone, la méthyl-bêta-naphtylcétone, la benzylacétone, le benzophénone, la para-hydroxyphénylbutanone, la cétone de céleri ou la livescone, la 6-isopropyldécahydro-2-naphtone, la diméthylocténone, la frescomenthe, le 4-(1éthoxyvinlye)-3,3,5,5-tétraméthylcyclohexanone, la méthylheptenone, la 2-(2-(4-méthyl-3-cyclohexène-1-yl)propyl)cyclopentanone, le 1-(p-menthène-6(2)yl)-1-propanone, le 4-(4-hydro-xy-3-méthoxyphényl)-2-butanone, le 2-acétyl-3,3-diméthylnorbornane, le 6,7-dihydro-1,1,2,3,3-pentaméthyl-4(5H)in-danone, le 4-damascol, le dulcinyle ou le cassion, le gelson, l'hexalone, l'isocyclemone E, la méthylcyclocitrone, la méthyllavendelcétone, l'orivone, le para-tertiaire butylcyclohexanone, la verdone, la delphone, le muscone, la néobuténone, la plicatone, la veloutone, le 2,4,4,7-tétraméthyl-6-oct-en-3-on, la tétramérane ou des composés de ceux-ci ainsi que
(b) du 2-amino-1,3-propanediol et/ou du 2-amino-1,3-propanediol substitué selon la formule (I) 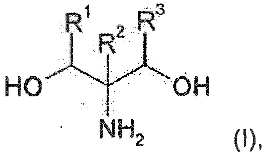 dans lequel dans cette formule les résidus R¹, R² ainsi que R³, respectivement indépendamment l'un de l'autre, représentent de l'hydrogène ou des résidus d'hydrocarbures, dans lequel les résidus R² très préférables sont outre de l'hydrogène notamment des résidus de méthyl, d'éthyl et d'hydroxyméthyl et (c) 5 % en poids à 50 % en poids en tensioactif, dans lequel le rapport du composant (a) sur le composant (b) s'élève de 25:1 à 1:5.

2. Agent selon la revendication 1, **caractérisé en ce que** le composant (a) et le composant (b) sont ajoutés séparément dans la matrice de l'agent de lavage, de nettoyage ou d'entretien.

3. Agent selon l'une quelconque des revendications 1 ou 2, **caractérisé en ce que** le composant (b) est présent dans l'agent dans des quantités de 0,001 % en poids à 3 % en poids, le % en poids étant rapporté à l'ensemble de l'agent.

4. Agent selon l'une quelconque des revendications 1 à 3, **caractérisé en ce que** qu'il contient 0,001 % en poids à 3 % en poids d'aldéhyde parfumé et/ou de cétone parfumée, le % en poids étant rapporté à l'ensemble de l'agent.

5. Agent selon l'une quelconque des revendications 1 à 4, **caractérisé en ce que** la quantité totale de substance aromatique de l'agent s'élève de 0,01 à 10 % en poids, le % en poids étant rapporté à l'ensemble de l'agent.

6. Agent selon l'une quelconque des revendications 1 à 5, **caractérisé en ce qu'**il se présente sous forme solide, de préférence sous forme de poudre.

7. Agent selon l'une quelconque des revendications 1 à 6, **caractérisé en ce qu'**il se présente sous forme liquide, de préférence sous forme de gel.

8. Agent selon l'une quelconque des revendications 1 à 7, **caractérisé en ce qu'**il contient au moins 5 % en poids, de préférence au moins 8 % en poids, notamment au moins 10 % en poids en tensioactif, notamment en tensioactif anionique et/ou tensioactif non-ionique, le % en poids étant rapporté à l'ensemble de l'agent.

9. Procédé de nettoyage des textiles ou de conditionnement, lors duquel le textile à nettoyer est soumis à un lavage à l'aide d'un agent de lavage, de nettoyage ou d'entretien selon l'une quelconque des revendications 1 à 8, notamment dans une machine à laver automatique.

10. Utilisation d'un agent de lavage, de nettoyage ou d'entretien selon l'une quelconque des revendications 1 à 8 pour le nettoyage et/ou le conditionnement de tissus textiles, notamment dans une machine à laver automatique.

11. Utilisation de 2-amino-1,3-propanediol et ou de 2-amino-1,3-propanediol substitué selon la formule (I) 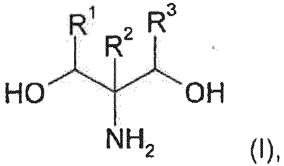 dans laquelle dans cette formule les résidus R¹, R² ainsi que R³, respectivement indépendamment l'un de l'autre, représentent de l'hydrogène ou des résidus d'hydrocarbures, en tant que composants ajoutés séparément dans les agents de lavage, de nettoyage ou d'entretien contenant des substances aromatiques, lesquelles contiennent des cétones et/ou des aldéhydes parfumés, pour le prolongement de l'effet parfumé de l'agent de lavage, de nettoyage ou d'entretien.

12. Utilisation de 2-amino-1,3-propanediol et ou de 2-amino-1,3-propanediol substitué selon la formule (I) 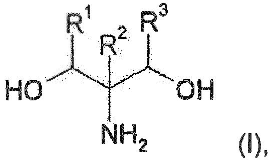 dans laquelle dans cette formule les résidus R¹, R² ainsi que R³, respectivement indépendamment l'un de l'autre, représentent de l'hydrogène ou des résidus d'hydrocarbures, en tant que composants ajoutés séparément dans les agents de lavage, de nettoyage ou d'entretien, lesquels contiennent des cétones et/ou des aldéhydes parfumés, afin d'obtenir un parfum de fraîcheur longue durée lors de l'utilisation de l'agent de lavage, de nettoyage ou d'entretien.
